# EUROPEAN PATENT APPLICATION

(11) **EP 4 661 011 A1**
(43) Date of publication of application: **10.12.2025**
(21) Application number: 24759425.2
(22) Date of filing: 04.01.2024
(51) Int. Cl.: G16B 15/30, G16C 10/00

(54) **TARGET DISCOVERY METHOD BASED ON METADYNAMICS, AND RELATED APPARATUS**

(30) Priority: 23.02.2023 CN 202310158877; 21.04.2023 CN 202310436968
(71) Applicant: Huawei Cloud Computing Technologies Co., Ltd., Guiyang, Guizhou 550025 (CN); Peking University, Beijing 100871 (CN)
(72) Inventor: HAN, Xu, Beijing 100871 (CN); QIU, Jing, Guiyang, Guizhou 550025 (CN); XIONG, Zhaoping, Guiyang, Guizhou 550025 (CN); LIN, Xinyuan, Guiyang, Guizhou 550025 (CN); REN, Feixiao, Guiyang, Guizhou 550025 (CN); YANG, Lijiang, Beijing 100871 (CN); GAO, Yiqin, Beijing 100871 (CN); QIAO, Nan, Guiyang, Guizhou 550025 (CN)
(74) Representative: Goddar, Heinz J.
(86) International application number: PCT/CN2024/070604
(87) International publication number: WO 2024/174746

(57) **Abstract**

Embodiments of this application provide a metadynamics-based target discovery method and a related apparatus, and are applied to the fields of biology, medicine, and drug design. The method includes: performing dynamics simulation on a ligand structure and a receptor structure, to enable the ligand structure to traverse a surface of the receptor structure during the dynamics simulation; determining target information of binding sites of the ligand structure on the surface of the receptor structure; and outputting pocket information of the receptor structure based on sorting of the target information of the binding sites. In this process, a plurality of binding sites can be obtained through one traversal. In this way, a receptor conformation search range can be wider, a calculation amount is reduced, and binding site discovery efficiency is improved.

## Description

This application claims priorities to Chinese Patent Application No. 202310158877.2, filed with the China National Intellectual Property Administration on February 23, 2023 and entitled "METADYNAMICS-BASED CONFORMATION SEARCH METHOD AND RELATED APPARATUS", and to Chinese Patent Application No. 202310436968.8, filed with the China National Intellectual Property Administration on April 21, 2023 and entitled "METADYNAMICS-BASED TARGET DISCOVERY METHOD AND RELATED APPARATUS", both of which are incorporated herein by reference in their entireties.

### TECHNICAL FIELD

This application relates to the fields of biology, medicine, drug design, and the like, and in particular, to a metadynamics-based target discovery method and a related apparatus.

### BACKGROUND

"High investment, long cycle, high risk, and low success rate" are pain points of the conventional new drug research and development industry. New drug research and development has a cycle longer than 12 years, costs USD2.6 billion on average, and has a final failure rate higher than 90%. A drug discovery stage mainly includes disease selection, target discovery, hit compound discovery, lead compound optimization, and other processes. A target with good druggability can greatly increase a success rate of discovery of a micromolecular drug. There are currently two difficulties during target discovery: 1. For newly discovered protein, a target location needs to be determined. 2. In an experiment, it is easy to obtain a micromolecule bound to protein, but it is difficult to determine a specific binding site or binding pattern. Therefore, how to find a precise binding site and binding conformation is a problem that urgently needs to be resolved.

### SUMMARY

Embodiments of this application provide a metadynamics-based target discovery method and a related apparatus, to make a receptor conformation search range wider and improve binding site discovery efficiency.

**According to a first aspect,** an embodiment of this application provides a metadynamics-based target discovery method. The method includes:
performing dynamics simulation on a ligand structure and a receptor structure, where a bias potential is configured for the ligand structure during the dynamics simulation, the bias potential of the ligand structure enables the ligand structure to traverse a surface of the receptor structure during the dynamics simulation, a calculation model for the bias potential includes a bias coefficient expression, and the bias coefficient expression reflects a bias degree of a binding conformation obtained by binding the ligand structure to the receptor structure;
determining target information of binding sites of the ligand structure on the surface of the receptor structure, where the target information includes retention time or a bias coefficient, a bias coefficient of each binding site is calculated based on a target parameter and the bias coefficient expression, and the target parameter includes a parameter value generated by the ligand structure during the dynamics simulation; and
outputting pocket information of the receptor structure based on a sorting result of the target information of the binding sites.

In the foregoing method, a plurality of binding sites can be obtained through one traversal. In this way, a receptor conformation search range can be wider, a calculation amount is reduced, and binding site discovery efficiency is improved.

In a possible implementation, the method further includes:
outputting display information in a visual manner, where the display information includes one or two of a process of traversing the surface of the receptor structure by the ligand structure during the dynamics simulation, or a process of binding the ligand structure to the receptor structure to form the binding conformation.

In the foregoing method, a plurality of pieces of information are output in a visual manner, so that a user can subsequently optimize or adjust a related parameter.

In another possible implementation, outputting the pocket information of the receptor structure based on the sorting result of the target information of the binding sites includes:
using, as the pocket information of the receptor structure, binding sites corresponding to the first N pieces of target information in the sorting result of the target information of the binding sites, where N is a positive integer greater than or equal to 1.

In the foregoing method, in a pocket scoring mode in this solution, the binding sites corresponding to the first N pieces of target information are selected. Based on the sorting result, better pharmaceutical effect can be achieved after a ligand, for example, a drug molecule, is bound to a receptor, for example, target protein.

In still another possible implementation, before performing dynamics simulation on the ligand structure and the receptor structure, the method further includes:
determining a solvent accessible surface of the receptor structure;
extracting a plurality of discrete points from the solvent accessible surface; and
updating location coordinates of a first discrete point based on one or more heavy atoms closest to the first discrete point, where the first discrete point is any one of the plurality of discrete points.

In the foregoing method, during discretization of the surface of the receptor structure, a possible binding site on a protein surface can be determined, so that a conformation space search range of the receptor structure is wider.

In still another possible implementation, updating the location coordinates of the first discrete point based on the one or more heavy atoms closest to the first discrete point includes:
mapping the first discrete point to a first coordinate system, and determining a second coordinate system based on a mapped location, where the first coordinate system is a coordinate system constructed based on locations of the first M heavy atoms closest to the first discrete point, and M is a positive integer greater than or equal to 3; and
updating the location coordinates of the first discrete point based on the second coordinate system, where location coordinates of the binding sites are location coordinates of discrete points closest to the binding sites, and the location coordinates of the binding sites are used to determine bias potentials of the binding sites.

In the foregoing method, compared with mapping a discrete point by using location coordinates of a light atom, this solution can improve location coordinate precision of a discrete point.

In still another possible implementation, extracting the plurality of discrete points from the solvent accessible surface includes:
extracting the plurality of discrete points from the solvent accessible surface based on a density requirement.

In the foregoing method, in this solution, the user can select a density status of the plurality of discrete points in a more diversified manner according to an actual requirement of the user.

In still another possible implementation, the method further includes:
outputting the plurality of discrete points in a visual manner.

In the foregoing method, a dynamic discretization process on the surface of the receptor structure is output to the user, so that the user can determine reliability of a target discovery process based on a surface discretization status.

In still another possible implementation, the calculation model for the bias potential is used to constrain, by using the bias coefficient expression and the target parameter, an acting force applied to the ligand structure.

In still another possible implementation, the target parameter further includes a preset parameter, the parameter value generated during the dynamics simulation includes center-of-mass location coordinates of the ligand structure and location coordinates of the discrete points, and the preset parameter includes a height of a Gaussian peak, a Gaussian of full width at half maximum, and a metadynamics harmonic parameter.

In still another possible implementation, the target parameter further includes a confining potential parameter, the confining potential parameter is used to constrain a distance from the ligand structure to the surface of the receptor structure to be less than a preset threshold, and the confining potential parameter includes a spring constant and the distance from the ligand structure to the surface of the receptor structure.

In the foregoing method, a distance between a ligand and a receptor can be limited within a specific range based on an added confining potential, to prevent the ligand from leaving a surface of the receptor, so that the ligand structure is more tightly bound to the receptor structure.

In still another possible implementation, during the dynamics simulation, longer retention time of the ligand structure at the binding site indicates a larger bias coefficient of the binding site.

In still another possible implementation, the ligand structure includes a micromolecular ligand structure or a macromolecular ligand structure.

In still another possible implementation, the receptor structure includes a protein structure.

**According to a second aspect,** an embodiment of this application further provides a method for discretizing a surface of a receptor structure. The method includes:
determining a solvent accessible surface of a receptor structure;
extracting a plurality of discrete points from the solvent accessible surface; and
updating location coordinates of a first discrete point based on one or more heavy atoms closest to the first discrete point, where the first discrete point is any one of the plurality of discrete points.

In the foregoing method, during discretization of the surface of the receptor structure, a possible binding site on a protein surface can be determined, so that a conformation space search range of the receptor structure is wider.

In a possible implementation, updating the location coordinates of the first discrete point based on the one or more heavy atoms closest to the first discrete point includes:
mapping the first discrete point to a first coordinate system, and determining a second coordinate system based on a mapped location, where the first coordinate system is a coordinate system constructed based on locations of the first M heavy atoms closest to the first discrete point, and M is a positive integer greater than or equal to 3; and
updating the location coordinates of the first discrete point based on the second coordinate system, where the location coordinates of the first discrete point are used as location coordinates of a nearest binding site, and the binding site is a site formed by binding the receptor structure to the ligand structure.

In the foregoing method, compared with mapping a discrete point by using location coordinates of a light atom, this solution can improve location coordinate precision of a discrete point.

**According to a third aspect,** this application further provides a metadynamics-based target discovery apparatus. The target discovery apparatus can implement some or all of functions completed by an electronic device according to the first aspect. For example, functions of the target discovery apparatus may include functions in some or all of embodiments of the electronic device according to the first aspect of this application. The functions may be implemented by hardware, or may be implemented by hardware executing corresponding software. The hardware or the software includes one or more units or modules corresponding to the functions.

In an optional implementation, the target discovery apparatus includes a simulation unit, a determining unit, and an output unit.

The simulation unit is configured to perform dynamics simulation on a ligand structure and a receptor structure, where a bias potential is configured for the ligand structure during the dynamics simulation, the bias potential of the ligand structure enables the ligand structure to traverse a surface of the receptor structure during the dynamics simulation, a calculation model for the bias potential includes a bias coefficient expression, and the bias coefficient expression reflects a bias degree of a binding conformation obtained by binding the ligand structure to the receptor structure.

The determining unit is configured to determine target information of binding sites of the ligand structure on the surface of the receptor structure, where the target information includes retention time or a bias coefficient, a bias coefficient of each binding site is calculated based on a target parameter and the bias coefficient expression, and the target parameter includes a parameter value generated by the ligand structure during the dynamics simulation.

The output unit is configured to output pocket information of the receptor structure based on a sorting result of the target information of the binding sites.

In another optional implementation, the output unit is further configured to output display information in a visual manner, where the display information includes one or two of a process of traversing the surface of the receptor structure by the ligand structure during the dynamics simulation, or a process of binding the ligand structure to the receptor structure to form the binding conformation.

In still another optional implementation, in terms of outputting the pocket information of the receptor structure based on the sorting result of the target information of the binding sites, the output unit is specifically configured to:
use, as the pocket information of the receptor structure, binding sites corresponding to the first N pieces of target information in the sorting result of the target information of the binding sites, where N is a positive integer greater than or equal to 1.

In still another optional implementation, the apparatus further includes an extraction unit and an update unit.

The determining unit is further configured to determine a solvent accessible surface of the receptor structure.

The extraction unit is configured to extract a plurality of discrete points from the solvent accessible surface.

The update unit is configured to update location coordinates of a first discrete point based on one or more heavy atoms closest to the first discrete point, where the first discrete point is any one of the plurality of discrete points.

In still another optional implementation, in terms of updating the location coordinates of the first discrete point based on the one or more heavy atoms closest to the first discrete point, the update unit is specifically configured to:
map the first discrete point to a first coordinate system, and determine a second coordinate system based on a mapped location, where the first coordinate system is a coordinate system constructed based on locations of the first M heavy atoms closest to the first discrete point, and M is a positive integer greater than or equal to 3; and
update the location coordinates of the first discrete point based on the second coordinate system, where location coordinates of the binding sites are location coordinates of discrete points closest to the binding sites, and the location coordinates of the binding sites are used to determine bias potentials of the binding sites.

In still another optional implementation, in terms of extracting the plurality of discrete points from the solvent accessible surface, the extraction unit is specifically configured to:
extract the plurality of discrete points from the solvent accessible surface based on a density requirement.

In still another optional implementation, the output unit is further configured to output the plurality of discrete points in a visual manner.

In still another optional implementation, the calculation model for the bias potential is used to constrain, by using the bias coefficient expression and the target parameter, an acting force applied to the ligand structure.

In still another optional implementation, the target parameter further includes a preset parameter, the parameter value generated during the dynamics simulation includes center-of-mass location coordinates of the ligand structure and location coordinates of the discrete points, and the preset parameter includes a height of a Gaussian peak, a Gaussian of full width at half maximum, and a metadynamics harmonic parameter.

In still another optional implementation, the target parameter further includes a confining potential parameter, the confining potential parameter is used to constrain a distance from the ligand structure to the surface of the receptor structure to be less than a preset threshold, and the confining potential parameter includes a spring constant and the distance from the ligand structure to the surface of the receptor structure.

In still another optional implementation, during the dynamics simulation, longer retention time of the ligand structure at the binding site indicates a larger bias coefficient of the binding site.

In still another optional implementation, the ligand structure includes a micromolecular ligand structure or a macromolecular ligand structure.

In still another optional implementation, the receptor structure includes a protein structure.

**According to a fourth aspect,** this application further provides a discretization apparatus for a surface of a receptor structure. The discretization apparatus can implement some or all of functions of an electronic device according to the first aspect. For example, functions of the discretization apparatus may include functions in some or all of embodiments of the electronic device according to the first aspect of this application. The functions may be implemented by hardware, or may be implemented by hardware executing corresponding software. The hardware or the software includes one or more units or modules corresponding to the functions.

In an optional implementation, the discretization apparatus includes a determining unit, an extraction unit, and an update unit.

The determining unit is configured to determine a solvent accessible surface of a receptor structure.

The extraction unit is configured to extract a plurality of discrete points from the solvent accessible surface.

The determining unit is further configured to map the first discrete point to a first coordinate system, and determine a second coordinate system based on a mapped location, where the first coordinate system is a coordinate system constructed based on locations of the first M heavy atoms closest to the first discrete point, the first discrete point is any one of the plurality of discrete points, and M is a positive integer greater than or equal to 3.

The update unit is configured to update the location coordinates of the first discrete point based on the second coordinate system, where the location coordinates of the first discrete point are used as location coordinates of a nearest binding site, and the binding site is a site formed by binding the receptor structure to the ligand structure.

**According to a fifth aspect,** an embodiment of this application provides an electronic device. The electronic device includes a processor. When the processor invokes a computer program or computer instructions in a memory, the method described in any one of the implementations of the first aspect is performed, or the method described in any one of the implementations of the second aspect is performed.

Optionally, the electronic device further includes a communication interface. The communication interface is configured to receive and/or send data, and/or the communication interface is configured to provide input and/or output for the processor.

It should be noted that, in the foregoing embodiment, the processor (or referred to as a general-purpose processor) that performs the method by invoking the computer instructions is used as an example for description. During specific implementation, the processor may alternatively be a dedicated processor. In this case, computer instructions are already pre-loaded on the processor. Optionally, the processor may alternatively include both a dedicated processor and a general-purpose processor.

Optionally, the communication apparatus may further include a memory, and the memory may be configured to store a computer program or computer instructions. Further, the memory may be located outside the processor, or may be integrated with the memory.

**According to a sixth aspect,** this application provides a computer-readable storage medium, configured to store instructions. When the instructions are run by a computer, the method according to the first aspect or the second aspect is implemented.

**According to a seventh aspect,** this application further provides a computer program product including instructions. When the computer program product is run on a computer, the method according to the first aspect or the second aspect is implemented.

Optionally, the computer program product may be a software installation package or an image package. When the foregoing method needs to be used, the computer program product may be downloaded, and the computer program product is executed on a computing device.

For beneficial effects of the technical solutions provided in the third aspect to the seventh aspect of this application, refer to the beneficial effects of the technical solutions in the first aspect and the second aspect. Details are not described herein again.

### BRIEF DESCRIPTION OF DRAWINGS

FIG. 1 is a diagram of a structure of an electronic device 10 according to an embodiment of this application;
FIG. 2 is a schematic flowchart of a metadynamics-based target discovery method according to an embodiment of this application;
FIG. 3 is a diagram of location coordinates of a discrete point according to an embodiment of this application;
FIG. 4 is a diagram of a retention location of a ligand structure on a surface of a receptor structure according to an embodiment of this application;
FIG. 5A is a diagram of performing conventional molecular dynamics simulation by using Anton according to an embodiment of this application;
FIG. 5B is a diagram of molecular dynamics simulation with shorter time according to an embodiment of this application;
FIG. 6 is a diagram of a binding conformation according to an embodiment of this application;
FIG. 7A is a diagram of a compound structure according to an embodiment of this application;
FIG. 7B is a diagram of a change in an RMSD of a receptor structure during molecular dynamics simulation according to an embodiment of this application;
FIG. 8 is a diagram of a structure of a metadynamics-based target discovery apparatus 80 according to an embodiment of this application; and
FIG. 9 is a diagram of a structure of a discretization apparatus 90 for a surface of a receptor structure according to an embodiment of this application.

### DESCRIPTION OF EMBODIMENTS

The following describes embodiments of this application with reference to accompanying drawings in embodiments of this application.

For ease of understanding, some concepts related to embodiments of this application are described below for reference by using examples. Details are as follows.

### 1. Molecular dynamics

The molecular dynamics is a comprehensive technology that combines physics, mathematics, and chemistry. The molecular dynamics is a molecular simulation method. In the method, motion of a molecular system is simulated mainly based on Newtonian mechanics, to extract a sample from a system including different states of the molecular system, calculate a configuration integral of the system, and further calculate a thermodynamic quantity and other macro properties of the system based on a configuration integral result. A basic idea of the molecular dynamics calculation is to extract a sample, for statistical calculation, from phase space based on natural motion of molecules after an initial motion status is assigned to the molecular system. A time step is a sampling interval. Therefore, selection of the time step is quite important for dynamics simulation. An excessively long time step causes severe collision between molecules and overflow of system data. An excessively short time step causes weakening of a capability of searching the phase space during simulation. Therefore, a selected time step is usually one tenth of a shortest motion cycle in each degree of freedom of the system. However, usually, vibration of each chemical bond has a shortest motion cycle in each degree of freedom. The motion does not affect calculation of some macro properties. Therefore, constraint dynamics that shields internal vibration of a molecule or other irrelevant motion emerges. Based on the constraint dynamics, a time step of molecular dynamics simulation can be effectively increased, to improve the capability of searching the phase space.

### 2. Drug target

A drug target is an action binding site of a drug in a body, and includes biological macromolecules such as a gene site, a receptor, an enzyme, an ion channel, and a nucleic acid. The key to modern new drug research and development is to find, determine, and prepare a drug screening target, namely, a molecular drug target. A primary task of new drug development is to select and determine a novel effective drug target. So far, approximately 500 treatment drug targets have been found. Most of the targets are receptors, especially G protein-coupled receptors (GPCR), and the targets also include action targets of enzymes, antibacterial drugs, antiviral drugs, and antiparasitic drugs. In a rational drug design (Rational Drug Design), a drug molecule may be designed based on a potential drug target, for example, an enzyme, a receptor, an ion channel, or a nucleic acid, disclosed in life science research, or based on chemical structure characteristics of an endogenous ligand and a natural substrate of the target, to discover a new drug that selectively acts on the target.

There are mainly two bottlenecks during new drug innovation: One bottleneck lies in determining and verification of a target biological macromolecule. The other bottleneck lies in design and discovery of a biologically active micromolecular drug. Discovery of a drug target is the source and foundation of new drug innovation. Discovery of a new drug usually becomes a breakthrough point for discovery of a series of new drugs.

### 3. Conformation

A conformation is a spatial arrangement formed by placing atoms around a single bond without changing a structure of a covalent bond in a molecule. When one conformation is changed to another conformation, breaking or re-formation of a covalent bond is not required. A change in a conformation does not cause a change in optical activity of a molecule.

### 4. Protein binding pocket

A protein binding pocket (Protein Binding Pocket) may also be referred to as a pocket for short, and is a cavity that is on a surface of or inside protein and that is suitable for binding to a ligand. A shape, a location, physicochemical characteristics, and a function of the pocket are determined by an amino acid residue around the pocket. Dynamics of the pocket is crucial to interaction specificity of protein. Flexibility (flexibility) and mobility (mobility) of a protein structure allow opening, closing, and adaptation of the binding pocket, to adjust a ligand binding process and perform a specific function of protein. This means that internal movement of protein needs to be considered during prediction of binding performance and design of a new ligand.

The descriptions of the foregoing related concepts may be applied to the following embodiments.

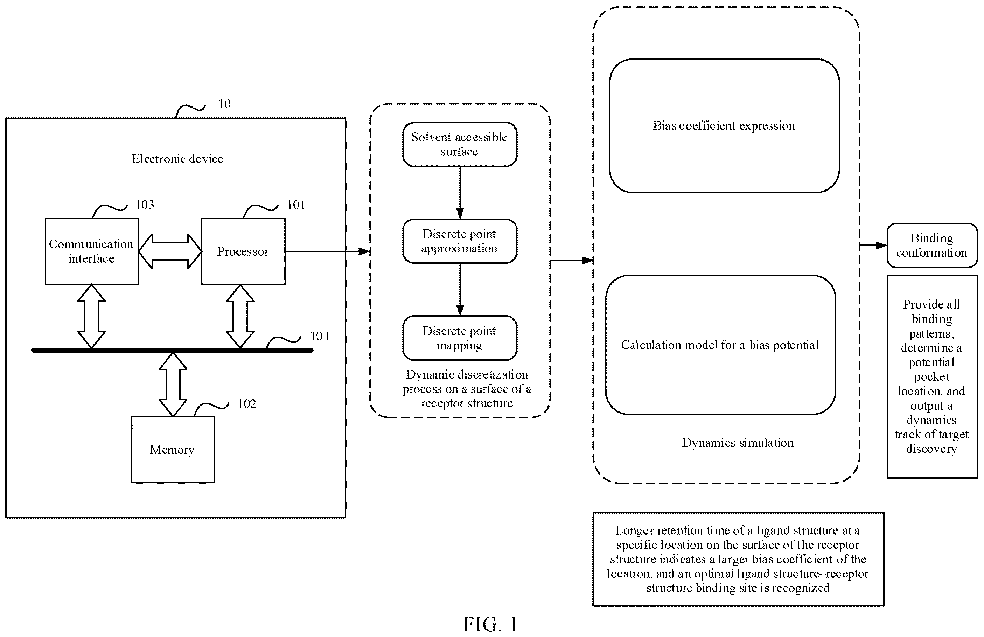

FIG. 1 is a diagram of a structure of an electronic device 10 according to an embodiment of this application. The electronic device 10 is configured to implement a dynamics-based target discovery method. The electronic device 10 may be a locally deployed device or a cloud server deployed on a cloud. The electronic device 10 may be a single device or a distributed system including a plurality of devices. The electronic device 10 may include a processor 101, and optionally, may further include at least one memory 102. Further, optionally, the electronic device 10 may further include a communication interface 103. Furthermore, optionally, the electronic device 10 may further include a bus 104. The processor 101, the memory 102, and the communication interface 103 are connected through the bus 104.

The processor 101 is a module for performing an arithmetic operation and/or a logic operation, and may be specifically one or a combination of a plurality of processing modules such as a central processing unit (Central Processing Unit, CPU), a graphics processing unit (Graphics Processing Unit, GPU), a microprocessor unit (Microprocessor Unit, MPU), an application-specific integrated circuit (Application-Specific Integrated Circuit, ASIC), a field programmable gate array (Field Programmable Gate Array, FPGA), a complex programmable logic device (Complex Programmable Logic Device, CPLD), a coprocessor (assisting the central processing unit in corresponding processing and application), and a microcontroller unit (Microcontroller Unit, MCU).

The memory 102 is configured to provide storage space, and the storage space may store data such as an operating system and a computer program. The memory 102 may be one or a combination of a random access memory (Random Access Memory, RAM), a read-only memory (Read-only Memory, ROM), an erasable programmable read-only memory (Erasable Programmable Read-only Memory, EPROM), a compact disc read-only memory (Compact Disc Read-only Memory, CD-ROM), and the like.

The communication interface 103 may be configured to provide information input or output for the at least one processor; and/or the communication interface 103 may be configured to receive data sent from the outside and/or send data to the outside. The communication interface 103 may be an interface of a wired link, including an Ethernet cable or the like; or may be an interface of a wireless link (Wi-Fi, Bluetooth, universal wireless transmission, an in-vehicle short-range communication technology, or the like). Optionally, the communication interface 103 may further include a transmitter (for example, a radio frequency transmitter or an antenna), a receiver, or the like that is coupled to the interface.

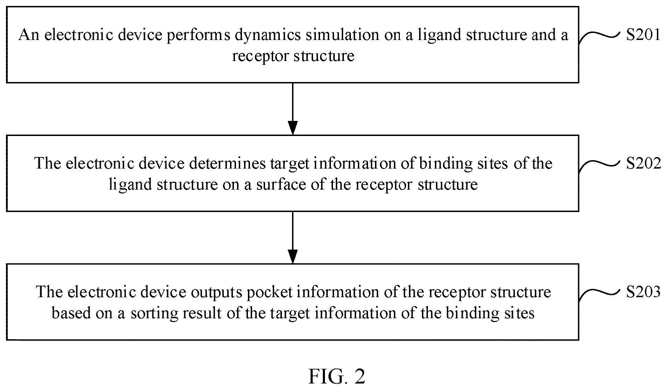

The processor 101 in the electronic device 10 is configured to perform a method described in an embodiment of FIG. 2. For example, the processor 101 in the electronic device 10 invokes a computer program, and may specifically perform the following operation:
performing a dynamic discretization operation on a surface of a receptor structure, where the dynamic discretization operation specifically includes three steps: calculating a solvent accessible surface of the receptor structure, fitting the solvent accessible surface based on a discrete point, and mapping the discrete point.

After completing the dynamic discretization operation, the processor 101 is further configured to perform dynamics simulation on the receptor structure and a ligand structure, to enable the ligand structure to traverse the surface of the receptor structure during the dynamics simulation.

Then the processor 101 determines target information of binding sites of the ligand structure on the surface of the receptor structure, and finally outputs pocket information of the receptor structure based on a sorting result of the target information of the binding sites.

In the foregoing operations, a more accurate binding site and a more accurate binding conformation can be found within shorter dynamics simulation time.

Optionally, the processor 101 may be a processor specially configured to perform the method (for ease of differentiation, the processor is referred to as a dedicated processor), or may be a processor that performs the method by invoking a computer program, for example, a general-purpose processor. In this case, the memory 102 is needed for storing the computer program invoked by the processor. Optionally, the at least one processor may alternatively include both a dedicated processor and a general-purpose processor.

FIG. 2 is a schematic flowchart of a metadynamics-based target discovery method according to an embodiment of this application. Optionally, the target discovery method may be applied to the foregoing electronic device for implementation, for example, the electronic device shown in FIG. 1.

The target discovery method includes but is not limited to the following step S201 to step S203. It should be understood that, herein, for ease of description, a sequence of step S201 to step S203 is used for description, but this is not intended to limit execution to the foregoing sequence. An execution sequence, execution time, a quantity of times of execution, or the like of one or more of the foregoing steps is not limited in this embodiment of this application. Another step may alternatively be performed between, before, or after these steps as needed.

**Step S201:** The electronic device performs dynamics simulation on a ligand structure and a receptor structure.

Specifically, when effective sampling needs to be performed on structural dynamics within a limited time scale, an enhanced sampling method, for example, metadynamics, may be used. The metadynamics is a method for improving sampling efficiency by introducing an additional bias potential (or force) and applying the bias potential (or force) to specific degrees of freedom. In the metadynamics method, effective simulation coverage time is increased, so that detailed sampling can be performed. A historical related bias (for example, a degree of freedom) is added to a system. The degree of freedom is usually referred to as a collective variable (Collective Variable, CV), and is usually a function of a point in state space and may be used for distinguishing between two or more thermodynamic states that need to be studied. The metadynamics, as a carefully selected CV function, enables sampling of a complex free energy graph. In this way, the electronic device does not re-access a previous sampling area, and is forced to evade a stable free energy minimum value, to facilitate exploration of an entire free energy landscape.

Therefore, to effectively perform sampling on structural dynamics within a limited time scale, during the dynamics simulation in this embodiment of this application, a bias potential is configured for the ligand structure, the bias potential of the ligand structure enables the ligand structure to traverse a surface of the receptor structure during the dynamics simulation, a calculation model for the bias potential includes a bias coefficient expression, and the bias coefficient expression reflects a bias degree of a binding conformation obtained by binding the ligand structure to the receptor structure. Therefore, according to the method in this embodiment of this application, traversing a protein surface by a micromolecule can be accelerated, so that a plurality of binding sites can be obtained through one traversal. This eliminates a problem, in conventional molecular dynamics simulation, that a time scale is long and only a single binding site can be found, and greatly improves binding site discovery efficiency.

In this embodiment of this application, location coordinates of a binding site need to be used for calculating or determining a bias potential of the binding site. Location coordinates of each binding site may be determined based on location coordinates of a discrete point. Therefore, optionally, the electronic device may first perform dynamic discretization on the surface of the receptor structure when performing dynamics simulation on the ligand structure and the receptor structure. Details are as follows.

First, the electronic device determines a solvent accessible surface of the receptor structure. Because the surface of the receptor structure is in a quite irregular shape, a first step of analyzing characteristics of the surface of the receptor structure is to define the surface of the receptor structure. During folding of the receptor structure, a hydrophobic amino acid is likely to be buried in a molecule. To quantitatively describe a burial status of the hydrophobic amino acid in the receptor structure, the solvent accessible surface is introduced. The solvent accessible surface is a "hypothetical curved surface", which is a track that a probe molecule passes when the probe molecule rolls on the surface of the receptor structure.

Then a plurality of discrete points are extracted from the solvent accessible surface. For example, the plurality of discrete points include a discrete point 1, a discrete point 2, a discrete point 3, ..., and a discrete point n. Optionally, the plurality of discrete points may be extracted from the solvent accessible surface based on a density requirement.

Usually, in different application scenarios or for different receptor structures or ligand structures, a density requirement for discrete points varies. Even in a same application scenario or for a same receptor structure or ligand structure, discrete points with different densities may be used. Therefore, in this application, corresponding configuration is performed, to enable a user to extract discrete points with corresponding densities according to an actual requirement. For example, if a density requirement of the user for a plurality of discrete points is being dense, the plurality of discrete points may be extracted from the solvent accessible surface based on a first density; or if a density requirement of the user for a plurality of discrete points is being sparse, the plurality of discrete points may be extracted from the solvent accessible surface based on a second density, where the second density is less than the first density. Therefore, in this embodiment of this application, the user captures discrete points in a more targeted manner.

In an optional implementation, after the plurality of discrete points are captured, the plurality of discrete points are output (for example, displayed) in a visual manner, and may be output as a picture, an animation, a table, text, or the like. In this solution, a dynamic discretization process on the surface of the receptor structure is output to the user, so that the user can determine reliability of a target discovery process based on a surface discretization status.

Then location coordinates of a first discrete point are updated based on one or more heavy atoms closest to the first discrete point. The location coordinates of the first discrete point may be updated in the following manner.

First, the first discrete point is mapped to a first coordinate system, and a second coordinate system is determined based on a mapped location, where the first discrete point is any one of the plurality of discrete points. To be specific, descriptions of characteristics of the first discrete point in this application document are merely an example, and actually, each of the plurality of discrete points has same characteristics as those of the first discrete point.

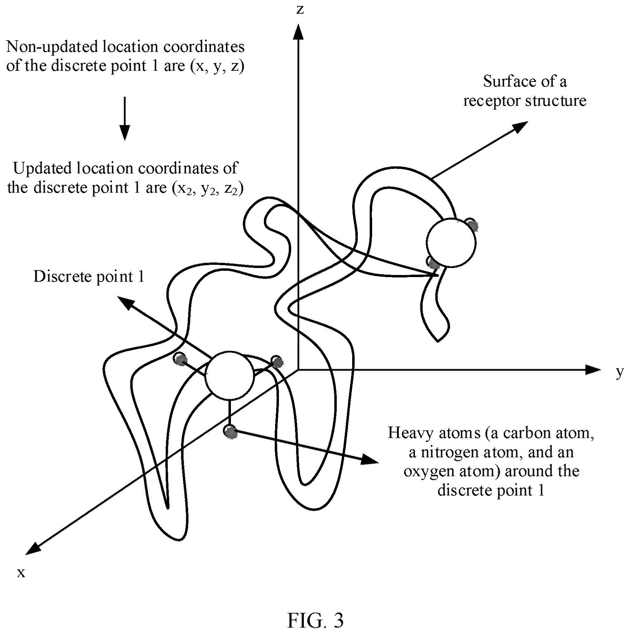

Then the location coordinates of the first discrete point are updated based on the second coordinate system. After a spatial conformation of the receptor structure is updated, there is no metadynamics equation for updating coordinates of a discrete point on the surface of the receptor structure. Therefore, the discrete point is not actively updated with the update of the spatial conformation of the receptor structure. FIG. 3 is a diagram of location coordinates of a discrete point according to an embodiment of this application. In this case, in this application, location coordinates of a discrete point on the surface are represented by location coordinates of the first M heavy atoms closest to the discrete point. The discrete point on the surface of the receptor structure can be updated with a subsequent change in the receptor structure only after mapping is performed once. It is assumed that M is a positive integer equal to 3. A discrete point 1 is used below as an example for description. For example, there are three heavy atoms closest to the discrete point 1: a carbon atom, a nitrogen atom, and an oxygen atom. A first coordinate system (x, y, z) (for example, x=1, y=2, and z=3) is formed based on the three heavy atoms. Then the discrete point 1 is mapped to the first coordinate system (x, y, z). When the first coordinate system (x, y, z) changes, a second coordinate system (x₂, y₂, z₂) (for example, x₂=2, y₂=1, and z₂=5) is determined based on an updated first coordinate system (x₁, y₁, z₁) obtained through mapping. In this case, the second coordinate system (x₂, y₂, z₂) may be the updated first coordinate system (x₁, y₁, z₁). To be specific, each time a coordinate system formed by the heavy atoms is updated, location coordinates of the discrete point 1 are also updated correspondingly. It should be noted that a manner of updating location coordinates of another discrete point is the same as the principle of the manner of updating the location coordinates of the discrete point 1. Details are not described herein again.

In an optional implementation, M in this solution may alternatively be a positive integer greater than 3. For example, when M is 5, there are five heavy atoms closest to the discrete point 1, and the electronic device constructs a five-dimensional coordinate system based on the five heavy atoms. For another example, when M is 5, there are five heavy atoms closest to the discrete point 1. However, if three heavy atoms are on a straight line, the electronic device constructs a three-dimensional coordinate system based on the five heavy atoms.

In an optional implementation, location coordinates of each binding site are location coordinates of a discrete point closest to the binding site.

That the location coordinates of each binding site are location coordinates of a discrete point closest to the binding site includes but is not limited to the following cases: The location coordinates of each binding site are determined based on location coordinates of a plurality of discrete points closest to the binding site, or the location coordinates of each binding site are determined based on location coordinates of one discrete point closest to the binding site or location coordinates of a discrete point at which the binding site is located.

**Case 1:** The location coordinates of each binding site are determined based on location coordinates of a plurality of discrete points closest to the binding site. Specific content is as follows:
Specifically, for example, if the ligand structure is migrated to locations of a plurality of discrete points (for example, the plurality of discrete points currently include a discrete point 1, a discrete point 2, and a discrete point 3 that are closest to a binding site A) on the surface of the receptor structure and is then bound to the plurality of discrete points to obtain the binding site A, the discrete point 1, the discrete point 2, and the discrete point 3 are near a location of the binding site A. In this case, location coordinates of the binding site A may be represented by average location coordinates of a plurality of discrete points closest to the binding site A. To be specific, the location coordinates of the binding site A are an average value of location coordinates of three discrete points near the binding site A: the discrete point 1, the discrete point 2, and the discrete point 3; and may be referred to as average location coordinates. It should be noted that this case may be applied to a plurality of scenarios, for example, a scenario in which a plurality of discrete points are densely distributed.

**Case 2:** The location coordinates of each binding site are determined based on location coordinates of one discrete point closest to the binding site. Specific content is as follows:
Specifically, for example, if the ligand structure is migrated to a location near a discrete point 4 on the surface of the receptor structure and is then bound to the discrete point 4 to obtain a binding site B, the binding site B is at a location closest to the discrete point 4. In this case, location coordinates of the binding site B may be represented by location coordinates of the discrete point 4. To be specific, the location coordinates of the binding site B are determined based on the location coordinates of the discrete point 4 closest to the binding site B. It should be noted that this case may be applied to a plurality of scenarios, for example, a scenario in which a plurality of discrete points are sparsely distributed.

**Case 3:** The location coordinates of each binding site are determined based on location coordinates of a discrete point at which the binding site is located. Specific content is as follows:
Specifically, for example, if the ligand structure is migrated to a location of a discrete point 5 on the surface of the receptor structure and is then bound to the discrete point 5 to obtain a binding site C, the binding site C is at the location of the discrete point 5. In this case, location coordinates of the binding site C may be represented by location coordinates of the discrete point 5. To be specific, the location coordinates of the binding site C are the location coordinates of the discrete point 5 at which the binding site C is located. It should be noted that this case may be applied to a plurality of scenarios, for example, a scenario in which a plurality of discrete points are sparsely distributed. In this embodiment of this application, in one solution, the ligand structure includes a micromolecular ligand structure; and in another solution, the ligand structure includes a macromolecular ligand structure. Optionally, in this embodiment of this application, the receptor structure includes a protein structure.

**Step S202:** The electronic device determines target information of binding sites of the ligand structure on the surface of the receptor structure.

The target information includes retention time or a bias coefficient. A bias coefficient of each binding site is calculated based on a target parameter and the bias coefficient expression. The target parameter includes a parameter value generated by the ligand structure during the dynamics simulation. For example, the parameter value generated by the ligand structure during the dynamics simulation may include one or more of coordinates rᵢ of an i^{th} discrete point and center-of-mass coordinates rₜ of the ligand structure within retention time, that is, a time period t.

Optionally, the target parameter further includes a preset parameter for which a user has preset a fixed value. The preset parameter may include one or more of a height/width ω of a Gaussian peak, a Gaussian of full width at half maximum *σ*, and a metadynamics harmonic parameter γ.

For ease of understanding, one bias coefficient expression is provided below as an example. Optionally, the bias coefficient expression is as follows: *α*ᵢ=∑ₜωe^{-(*r*ₜ-*r*ᵢ)2/2*_{σ}*2}*σ*e^{-*α*ᵢ(t)/γ}, where *α*ᵢ represents a bias coefficient of the ligand structure at a location i, ω represents a height/width of a Gaussian peak, rₜ represents center-of-mass coordinates of the ligand structure within retention time, that is, a time period t, rᵢ represents coordinates of an i^{th} discrete point, *σ* represents a Gaussian of full width at half maximum, and γ represents a metadynamics harmonic parameter. Further, because the calculation model for the bias potential includes the bias coefficient expression, optionally, the calculation model for the bias potential may be set as follows: U_{bias}=∑ᵢ*α*_{i,shift}e^{-(*r*-*r*ᵢ)2/2*σ*2}, and *α*_{i,shift}=*α*ᵢ-*α*ₖ, where U_{bias} represents the bias potential, r represents center-of-mass coordinates of the ligand structure during a dynamics process, and *a*ₖ represents a bias coefficient of the ligand structure at a location k. The calculation model for the bias potential is used to constrain, by using the bias coefficient expression and the target parameter, an acting force applied to the ligand structure.

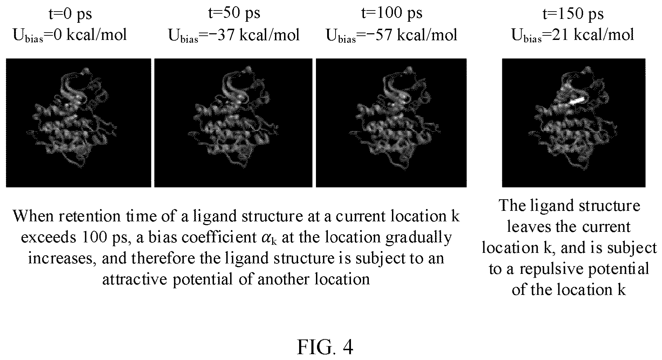

With reference to the calculation model for the bias potential, it can be learned that, when the ligand structure does not move, that is, t=0 ps, U_{bias}=0 kcal/mol. Further, for example, when t=50 ps, U_{bias}=-37 kcal/mol; when t=100 ps, U_{bias}=-57 kcal/mol; or when t=150 ps, U_{bias}=21 kcal/mol. FIG. 4 is a diagram of a retention location of a ligand structure on a surface of a receptor structure according to an embodiment of this application. When retention time of the ligand structure at a current location k exceeds 100 ps, a bias coefficient *α*ₖ at the location gradually increases. Therefore, the ligand structure is subject to an attractive potential of another location. When retention time of the ligand structure at a current location is 150 ps, the ligand structure leaves the current location k, and is subject to a repulsive potential of the location k. A dynamic migration process of the ligand structure on the surface of the receptor structure can be implemented based on a bias degree of the bias potential.

In an optional implementation, the target parameter further includes a confining potential parameter. A calculation model, obtained by adding a confining potential, for the bias potential is as follows: U=U_{bias}+k(|r-rₖ|-d)², where k(|r-rₖ|-d)² represents a calculation model for the confining potential. A parameter in the calculation model for the confining potential is the confining potential parameter. The confining potential parameter includes a spring constant k and a distance d from the ligand structure to the surface of the receptor structure. rₖ represents coordinates of a k^{th} discrete point. The confining potential is used to constrain the distance from the ligand structure to the surface of the receptor structure to be less than a preset threshold (for example, if the preset threshold is 2, the distance from the ligand structure to the surface of the receptor structure being 1.8 indicates that the ligand structure has not left the surface of the receptor structure).

In an optional implementation, during the dynamics simulation, retention time of the ligand structure at a binding site is strongly associated with a bias coefficient of the binding site. For example, longer retention time of the ligand structure at a binding site indicates a larger bias coefficient of the binding site.

**Step S203:** The electronic device outputs pocket information of the receptor structure based on a sorting result of the target information of the binding sites.

In an optional implementation, binding sites corresponding to the first N pieces of target information in the sorting result of the target information of the binding sites are used as the pocket information of the receptor structure, where N is a positive integer greater than or equal to 1. In a pocket scoring mode in this solution, the binding sites corresponding to the first N pieces of target information are selected. Based on the sorting result, better pharmaceutical effect can be achieved after a ligand, for example, a drug molecule, is bound to a receptor, for example protein.

Specifically, descriptions are provided by using an example in which binding sites whose target information is ranked in the first N positions is used as the pocket information of the receptor structure. A manner of determining the pocket information of the receptor structure by the electronic device includes but is not limited to the following two manners: using binding sites whose retention time is ranked in the first N positions as the pocket information of the receptor structure, or using binding sites whose bias coefficients are ranked in the first N positions as the pocket information of the receptor structure.

**Manner 1:** Use binding sites whose retention time is ranked in the first N positions as the pocket information of the receptor structure. Specific content is as follows:
**Example 1:** When N is equal to 1, in a process in which the ligand structure accelerates traversal of the surface of the receptor structure, the electronic device obtains data information of retention time of a plurality of binding sites whose retention time is ranked in the first N positions. The pocket information of the receptor structure may be an optimal binding site obtained by the electronic device by comprehensively considering a plurality of pieces of retention time corresponding to the ligand structure at the plurality of binding sites (for example, the following retention time is obtained in total by comprehensively considering the plurality of pieces of retention time corresponding to the ligand structure at the plurality of binding sites: 0.1 ns, 0.2 ns, 0.35 ns, 0.5 ns, and 1 ns, where 1 ns is the longest retention time). Therefore, a binding site at which retention time of the ligand structure is 1 ns may be selected as the pocket information of the receptor structure.

**Example 2:** When N is equal to 3, the pocket information of the receptor structure may be binding sites that correspond to retention time ranked in the first three positions and that are obtained by the electronic device by comprehensively considering a plurality of pieces of retention time corresponding to the ligand structure at a plurality of binding sites (for example, the following retention time is obtained in total by comprehensively considering the plurality of pieces of retention time corresponding to the ligand structure at the plurality of binding sites: 0.1 ns, 0.2 ns, 0.35 ns, 0.5 ns, and 1 ns, where 1 ns, 0.5 ns, and 0.35 ns are the retention time ranked in the first three positions). Therefore, binding sites at which retention time of the ligand structure is 1 ns, 0.5 ns, and 0.35 ns may be selected as the pocket information of the receptor structure.

**Manner 2:** Use binding sites whose bias coefficients are ranked in the first N positions as the pocket information of the receptor structure. Specific content is as follows:
**Example 3:** When N is equal to 1, in a process in which the ligand structure accelerates traversal of the surface of the receptor structure, the electronic device obtains data information of bias coefficients of a plurality of binding sites whose bias coefficients are ranked in the first N positions. The pocket information of the receptor structure may be an optimal binding site obtained by the electronic device by comprehensively considering a plurality of bias coefficients corresponding to the ligand structure at the plurality of binding sites (for example, the following bias coefficients are obtained in total by comprehensively considering the plurality of bias coefficients corresponding to the ligand structure at the plurality of binding sites: 5.1, 5.04, 3.32, 6.23, and 2.17, where 6.23 is the largest bias coefficient). Therefore, a binding site at which a bias coefficient of the ligand structure is 6.23 may be selected as the pocket information of the receptor structure.
**Example 4:** When N is equal to 3, the pocket information of the receptor structure may be binding sites that correspond to retention time ranked in the first three positions and that are obtained by the electronic device by comprehensively considering a plurality of bias coefficients corresponding to the ligand structure at a plurality of binding sites (for example, the following bias coefficients are obtained in total by comprehensively considering the plurality of bias coefficients corresponding to the ligand structure at the plurality of binding sites: 5.1, 5.04, 3.32, 6.23, and 2.17, where 6.23, 5.1, and 5.04 are the bias coefficients ranked in the first three positions). Therefore, binding sites at which bias coefficients of the ligand structure are 6.23, 5.1, and 5.04 may be selected as the pocket information of the receptor structure.

In an optional implementation, display information is output (for example, displayed) in a visual manner, and may be output as a picture, an animation, a table, text, or the like. The display information includes one or two of a process of traversing the surface of the receptor structure by the ligand structure during the dynamics simulation, or a process of binding the ligand structure to the receptor structure to form the binding conformation. In this solution, a plurality of pieces of information are output in a visual manner, so that a user can subsequently optimize or adjust a related parameter.

In this embodiment of this application, during the dynamics simulation on the ligand structure and the receptor structure, a plurality of binding sites can be obtained through one traversal. In this way, a receptor conformation search range can be wider, a calculation amount is reduced, and binding site discovery efficiency is improved.

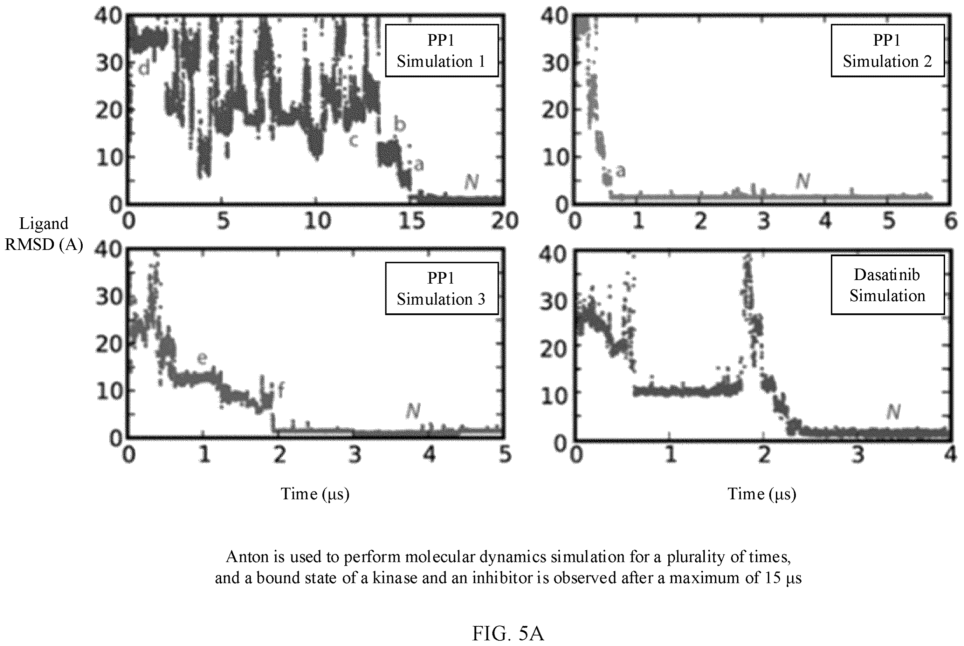

In the embodiment shown in FIG. 2, a process of performing, by the electronic device, target discovery based on metadynamics is described in detail. For ease of understanding, the following provides two specific cases of two example processes with reference to FIG. 5A, FIG. 5B, FIG. 6, FIG. 7A, and FIG. 7B.

An inhibitor (PP1) and kinase (Src) binding conformation search issue is used as an example to compare simulation time needed in embodiments of this application and simulation time needed in a conventional molecular dynamics method. Details are as follows.

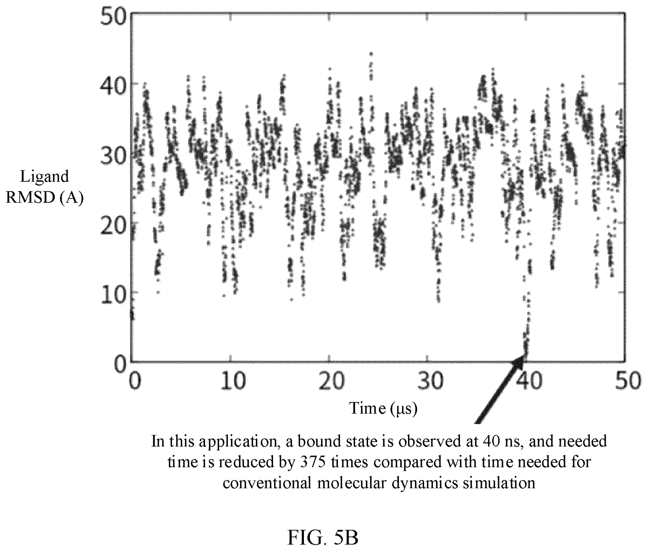

Case 1: FIG. 5A is a diagram of performing conventional molecular dynamics simulation by using a supercomputer Anton according to an embodiment of this application. As shown in FIG. 5A, the D. E. Shaw research group in the United States establishes the supercomputer Anton specially used for molecular dynamics simulation. Anton is used to perform molecular dynamics simulation for a plurality of times, and a bound state of a kinase and an inhibitor is observed after a maximum of 15 µs. The computing consumes huge computing power. FIG. 5B is a diagram of molecular dynamics simulation with shorter time according to an embodiment of this application. As shown in FIG. 5B, in this embodiment of this application, a ligand root mean square deviation (namely, ligand RMSD) changes with time t. In this application, a bound state is observed at 40 ns, and needed time is reduced by 375 times compared with time needed for conventional molecular dynamics simulation. The result indicates that, compared with the conventional molecular dynamics simulation, in this application, a binding conformation of a receptor structure and a ligand structure can be found within shorter dynamics simulation time.

A binding conformation of a compound structure (PDBID: 1u2s) and an SPP monomer structure (PDBID: 1s2o) is used as an example to compare flexibility of a receptor structure and a ligand structure in embodiments of this application and flexibility of a receptor structure and a ligand structure in a conventional molecular docking method. Details are as follows.

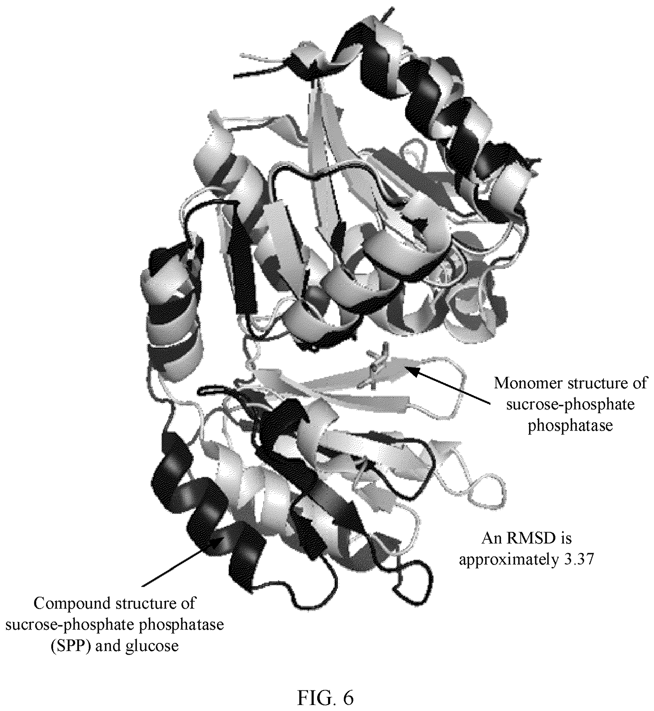

Case 2: FIG. 6 is a diagram of a binding conformation according to an embodiment of this application. As shown in FIG. 6, a compound structure (PDBID: 1u2s) greatly differs from an SPP monomer structure (PDBID: 1s2o), and an RMSD is approximately 3.37. The compound structure (PDBID: 1u2s) is a compound structure of sucrose-phosphate phosphatase (SPP) and glucose.

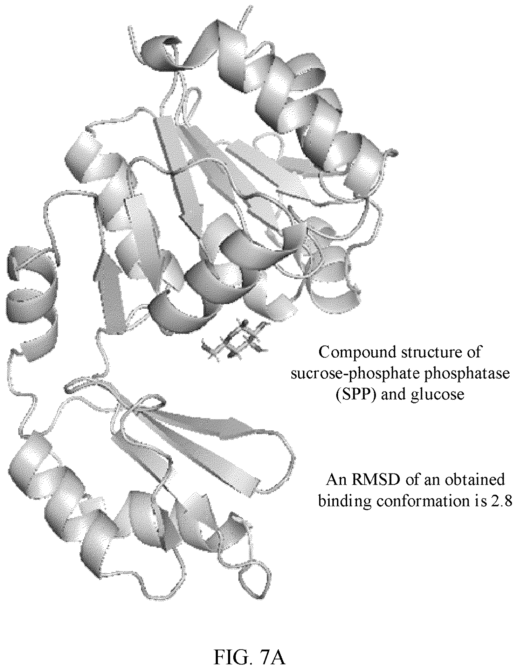

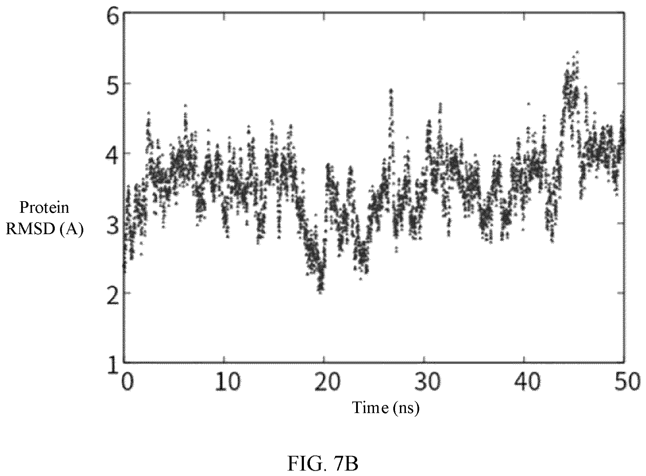

FIG. 7A is a diagram of a compound structure according to an embodiment of this application. As shown in FIG. 7A, in this application, starting from an SPP monomer structure, glucose is migrated on a surface of SPP, so that a result closer to an actual binding conformation result can be obtained through sampling. An RMSD of a binding conformation obtained in this application is 2.8. In addition, FIG. 7B is a diagram of a change in an RMSD of a receptor structure during molecular dynamics simulation according to an embodiment of this application. As shown in FIG. 7B, during the simulation, the RMSD of the receptor structure keeps changing. This indicates that the receptor structure is flexible during the molecular dynamics simulation in this application, so that sampling can be more fully performed on the receptor structure.

In this embodiment of this application, for a receptor structure whose structure greatly changes before and after binding, flexibility of both the receptor structure and a ligand structure may be considered in this application. Compared with the conventional molecular docking method, for a system in which a conformation in a bound state greatly differs from a conformation in a non-bound state, a result obtained in this application is closer to an actual binding conformation, and binding conformation search space is also wider.

The foregoing describes in detail the method in embodiments of this application. The following provides apparatuses in embodiments of this application.

It can be understood that, to implement the functions in the foregoing method embodiments, a plurality of apparatuses provided in embodiments of this application, for example, a target discovery apparatus, include a corresponding hardware structure or software module, a combination of a hardware structure and a software structure, or the like for performing the functions.

A person skilled in the art should be easily aware that, in combination with units and algorithm steps in the examples described in embodiments disclosed in this specification, embodiments of this application can be implemented by hardware or a combination of hardware and computer software. Whether a function is performed by hardware or hardware driven by computer software depends on particular applications and design constraints of the technical solutions. A person skilled in the art may implement the foregoing method embodiments by using different apparatus implementations in different use scenarios. It should not be considered that different implementations of the apparatus go beyond the scope of embodiments of this application.

In embodiments of this application, the apparatus may be divided into functional modules. For example, each functional module may be obtained through division based on each corresponding function, or two or more functions may be integrated into one processing module. The integrated module may be implemented in a form of hardware, or may be implemented in a form of a software functional module. It should be noted that division into the modules in embodiments of this application is an example, and is merely logical function division. During actual implementation, another division manner may be used.

For example, when the functional modules of the apparatus are divided in an integrated manner, several possible processing apparatuses are provided in this application.

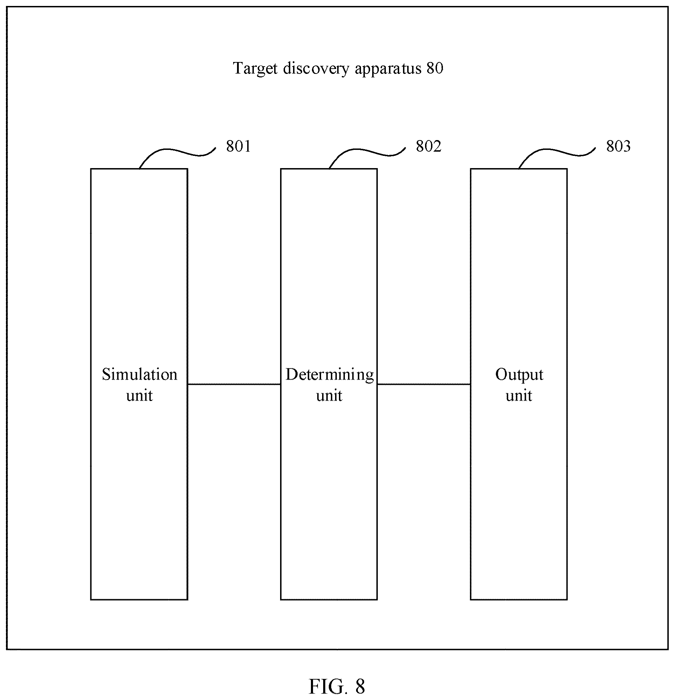

FIG. 8 is a diagram of a structure of a metadynamics-based target discovery apparatus 80 according to an embodiment of this application. The target discovery apparatus 80 may be the electronic device shown in FIG. 1 or a component in the electronic device, for example, a chip, a software module, or an integrated circuit. The target discovery apparatus 80 has a function of implementing the electronic device described in embodiments of this application. For example, the target discovery apparatus 80 includes a corresponding module, unit, or means (means) for the electronic device to perform the steps related to the electronic device described in embodiments of this application. The function, the unit, or the means (means) may be implemented by software, or may be implemented by hardware executing corresponding software, or may be implemented by a combination of software and hardware. For details, refer to the corresponding descriptions in the foregoing corresponding method embodiments.

In a possible design, the target discovery apparatus 80 includes a simulation unit 801, a determining unit 802, and an output unit 803.

The simulation unit 801 is configured to perform dynamics simulation on a ligand structure and a receptor structure, where a bias potential is configured for the ligand structure during the dynamics simulation, the bias potential of the ligand structure enables the ligand structure to traverse a surface of the receptor structure during the dynamics simulation, a calculation model for the bias potential includes a bias coefficient expression, and the bias coefficient expression reflects a bias degree of a binding conformation obtained by binding the ligand structure to the receptor structure.

The determining unit 802 is configured to determine target information of binding sites of the ligand structure on the surface of the receptor structure, where the target information includes retention time or a bias coefficient, a bias coefficient of each binding site is calculated based on a target parameter and the bias coefficient expression, and the target parameter includes a parameter value generated by the ligand structure during the dynamics simulation.

The output unit 803 is configured to output pocket information of the receptor structure based on a sorting result of the target information of the binding sites.

In an optional implementation, the output unit 803 is further configured to output display information in a visual manner, where the display information includes one or two of a process of traversing the surface of the receptor structure by the ligand structure during the dynamics simulation, or a process of binding the ligand structure to the receptor structure to form the binding conformation.

In another optional implementation, in terms of outputting the pocket information of the receptor structure based on the sorting result of the target information of the binding sites, the output unit 803 is specifically configured to:
use, as the pocket information of the receptor structure, binding sites corresponding to the first N pieces of target information in the sorting result of the target information of the binding sites, where N is a positive integer greater than or equal to 1.

In still another optional implementation, the apparatus further includes an extraction unit and an update unit.

The determining unit 802 is further configured to determine a solvent accessible surface of the receptor structure.

The extraction unit is configured to extract a plurality of discrete points from the solvent accessible surface.

The update unit is configured to update location coordinates of a first discrete point based on one or more heavy atoms closest to the first discrete point, where the first discrete point is any one of the plurality of discrete points.

In still another optional implementation, in terms of updating the location coordinates of the first discrete point based on the one or more heavy atoms closest to the first discrete point, the update unit is specifically configured to:
map the first discrete point to a first coordinate system, and determine a second coordinate system based on a mapped location, where the first coordinate system is a coordinate system constructed based on locations of the first M heavy atoms closest to the first discrete point, and M is a positive integer greater than or equal to 3; and
update the location coordinates of the first discrete point based on the second coordinate system, where location coordinates of the binding sites are location coordinates of discrete points closest to the binding sites, and the location coordinates of the binding sites are used to determine bias potentials of the binding sites.

In still another optional implementation, in terms of extracting the plurality of discrete points from the solvent accessible surface, the extraction unit is specifically configured to:
extract the plurality of discrete points from the solvent accessible surface based on a density requirement.

In still another optional implementation, the output unit is further configured to output the plurality of discrete points in a visual manner.

In still another optional implementation, the calculation model for the bias potential is used to constrain, by using the bias coefficient expression and the target parameter, an acting force applied to the ligand structure.

In still another optional implementation, the target parameter further includes a preset parameter, the parameter value generated during the dynamics simulation includes center-of-mass location coordinates of the ligand structure and location coordinates of the discrete points, and the preset parameter includes a height of a Gaussian peak, a Gaussian of full width at half maximum, and a metadynamics harmonic parameter.

In still another optional implementation, the target parameter further includes a confining potential parameter, the confining potential parameter is used to constrain a distance from the ligand structure to the surface of the receptor structure to be less than a preset threshold, and the confining potential parameter includes a spring constant and the distance from the ligand structure to the surface of the receptor structure.

In still another optional implementation, during the dynamics simulation, longer retention time of the ligand structure at the binding site indicates a larger bias coefficient of the binding site.

In still another optional implementation, the ligand structure includes a micromolecular ligand structure or a macromolecular ligand structure.

In still another optional implementation, the receptor structure includes a protein structure.

This embodiment of this application and the foregoing method embodiments are based on a same concept, and have same technical effect. For a specific principle, refer to the descriptions of the foregoing embodiments. Details are not described again.

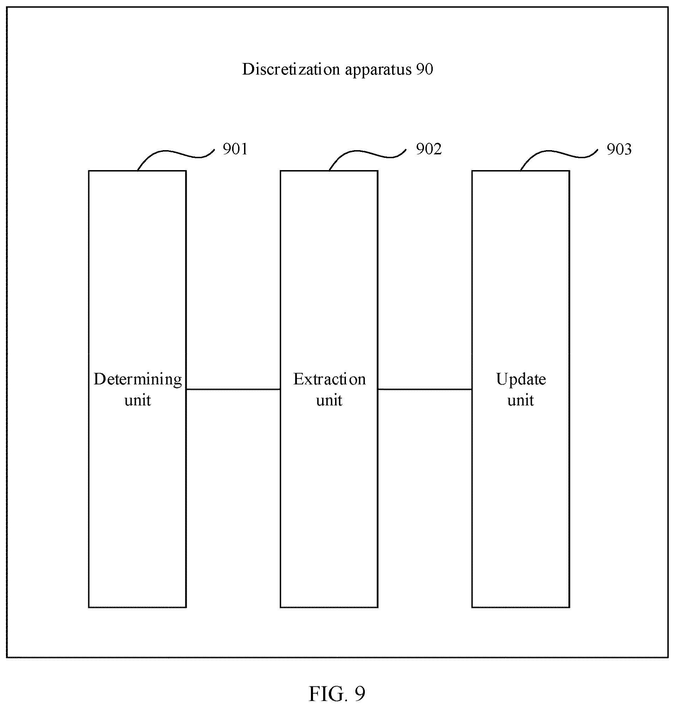

FIG. 9 is a diagram of a structure of a discretization apparatus 90 for a surface of a receptor structure according to an embodiment of this application. The discretization apparatus 90 may be the electronic device shown in FIG. 1 or a component in the electronic device, for example, a chip, a software module, or an integrated circuit. The discretization apparatus 90 has a function of implementing the electronic device described in embodiments of this application. For example, the discretization apparatus 90 includes a corresponding module, unit, or means (means) for the electronic device to perform the steps related to the electronic device described in embodiments of this application. The function, the unit, or the means (means) may be implemented by software, or may be implemented by hardware executing corresponding software, or may be implemented by a combination of software and hardware. For details, refer to the corresponding descriptions in the foregoing corresponding method embodiments.

In a possible design, the discretization apparatus 90 includes a determining unit 901, an extraction unit 902, and an update unit 903.

The determining unit 901 is configured to determine a solvent accessible surface of a receptor structure.

The extraction unit 902 is configured to extract a plurality of discrete points from the solvent accessible surface.

The update unit 903 is configured to update location coordinates of a first discrete point based on one or more heavy atoms closest to the first discrete point, where the first discrete point is any one of the plurality of discrete points.

In an optional implementation, in terms of updating the location coordinates of the first discrete point based on the one or more heavy atoms closest to the first discrete point, the update unit 903 is specifically configured to:
map the first discrete point to a first coordinate system, and determine a second coordinate system based on a mapped location, where the first coordinate system is a coordinate system constructed based on locations of the first M heavy atoms closest to the first discrete point, the first discrete point is any one of the plurality of discrete points, and M is a positive integer greater than or equal to 3; and
update the location coordinates of the first discrete point based on the second coordinate system, where the location coordinates of the first discrete point are used as location coordinates of a nearest binding site, and the binding site is a site formed by binding the receptor structure to the ligand structure.

This embodiment of this application and the foregoing method embodiments are based on a same concept, and have same technical effect. For a specific principle, refer to the descriptions of the foregoing embodiments. Details are not described again.

In embodiments of this application, unless otherwise specified or a logic conflict occurs, terms and/or descriptions in embodiments are consistent and may be mutually referenced, and technical features in different embodiments may be combined into a new embodiment based on an internal logical relationship between the technical features.

This application further provides a computer-readable storage medium. The computer-readable storage medium stores instructions. When the instructions are run on at least one processor, the foregoing communication method, for example, the method in FIG. 2, is implemented.

This application further provides a computer program product. The computer program product includes computer instructions. When the computer instructions are executed by a computing device, the foregoing communication method, for example, the method in FIG. 2, is implemented.

In embodiments of this application, the term "example", "for example", or the like is used to give an example, an illustration, or a description. Any embodiment or design scheme described with "example" or "for example" in this application should not be construed as being more preferred or advantageous than another embodiment or design scheme. To be precise, the term "example", "for example", or the like is intended to present a related concept in a specific manner.

In embodiments of this application, "at least one" means one or more, and "a plurality of" means two or more. "At least one of the following items (pieces)" or a similar expression thereof indicates any combination of the items, including one of the items (pieces) or any combination of a plurality of the items (pieces). For example, at least one of a, b, or c may indicate a, b, c, (a and b), (a and c), (b and c), or (a, b, and c), where a, b, and c may be in a singular form or a plural form. "And/or" describes an association relationship between associated objects and indicates that three relationships may exist. For example, A and/or B may indicate the following three cases: Only A exists, both A and B exist, and only B exists, where A and B may be singular or plural. The character "/" usually indicates an "or" relationship between the associated objects.

In addition, unless otherwise specified, ordinal numbers such as "first" and "second" in embodiments of this application are intended to distinguish between a plurality of objects, but not to limit an order, a time sequence, priorities, or importance of the plurality of objects. For example, a first device and a second device are merely intended for ease of description, but do not indicate that the first device and the second device have different structures, different importance, or the like. In some embodiments, the first device and the second device may alternatively be a same device.

Based on the context, the term "when" used in the foregoing embodiments may be interpreted as "if", "after", "in response to determining", or "in response to detecting". The foregoing descriptions are merely optional embodiments of this application, but are not intended to limit this application. Any modification, equivalent replacement, improvement, or the like made within the concept and principle of this application shall fall within the protection scope of this application.

A person of ordinary skill in the art can understand that all or some of the steps in the foregoing embodiments may be implemented by hardware or by a program instructing related hardware. The program may be stored in a computer-readable storage medium. The storage medium may be a read-only memory, a magnetic disk, a compact disc, or the like.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A metadynamics-based target discovery method, wherein the method comprises:
performing dynamics simulation on a ligand structure and a receptor structure, wherein a bias potential is configured for the ligand structure during the dynamics simulation, the bias potential of the ligand structure enables the ligand structure to traverse a surface of the receptor structure during the dynamics simulation, a calculation model for the bias potential comprises a bias coefficient expression, and the bias coefficient expression reflects a bias degree of a binding conformation obtained by binding the ligand structure to the receptor structure;
determining target information of binding sites of the ligand structure on the surface of the receptor structure, wherein the target information comprises retention time or a bias coefficient, a bias coefficient of each binding site is calculated based on a target parameter and the bias coefficient expression, and the target parameter comprises a parameter value generated by the ligand structure during the dynamics simulation; and
outputting pocket information of the receptor structure based on a sorting result of the target information of the binding sites.

2. The method according to claim 1, further comprising:
outputting display information in a visual manner, wherein the display information comprises one or two of a process of traversing the surface of the receptor structure by the ligand structure during the dynamics simulation, or a process of binding the ligand structure to the receptor structure to form the binding conformation.

3. The method according to claim 1 or 2, wherein outputting the pocket information of the receptor structure based on the sorting result of the target information of the binding sites comprises:
using, as the pocket information of the receptor structure, binding sites corresponding to the first N pieces of target information in the sorting result of the target information of the binding sites, wherein N is a positive integer greater than or equal to 1.

4. The method according to any one of claims 1 to 3, wherein before performing dynamics simulation on the ligand structure and the receptor structure, the method further comprises:
determining a solvent accessible surface of the receptor structure;
extracting a plurality of discrete points from the solvent accessible surface; and
updating location coordinates of a first discrete point based on one or more heavy atoms closest to the first discrete point, wherein the first discrete point is any one of the plurality of discrete points.

5. The method according to claim 4, wherein updating the location coordinates of the first discrete point based on the one or more heavy atoms closest to the first discrete point comprises:
mapping the first discrete point to a first coordinate system, and determining a second coordinate system based on a mapped location, wherein the first coordinate system is a coordinate system constructed based on locations of the first M heavy atoms closest to the first discrete point, and M is a positive integer greater than or equal to 3; and
updating the location coordinates of the first discrete point based on the second coordinate system, wherein location coordinates of the binding sites are location coordinates of discrete points closest to the binding sites, and the location coordinates of the binding sites are used to determine bias potentials of the binding sites.

6. The method according to claim 4 or 5, wherein extracting the plurality of discrete points from the solvent accessible surface comprises:
extracting the plurality of discrete points from the solvent accessible surface based on a density requirement.

7. The method according to any one of claims 4 to 6, further comprising:
outputting the plurality of discrete points in a visual manner.

8. The method according to any one of claims 1 to 7, wherein the calculation model for the bias potential is used to constrain, by using the bias coefficient expression and the target parameter, an acting force applied to the ligand structure.

9. The method according to claim 8, wherein the target parameter further comprises a preset parameter, the parameter value generated during the dynamics simulation comprises center-of-mass location coordinates of the ligand structure and location coordinates of the discrete points, and the preset parameter comprises a height of a Gaussian peak, a Gaussian of full width at half maximum, and a metadynamics harmonic parameter.

10. The method according to claim 8 or 9, wherein the target parameter further comprises a confining potential parameter, the confining potential parameter is used to constrain a distance from the ligand structure to the surface of the receptor structure to be less than a preset threshold, and the confining potential parameter comprises a spring constant and the distance from the ligand structure to the surface of the receptor structure.

11. The method according to any one of claims 1 to 10, wherein during the dynamics simulation, longer retention time of the ligand structure at the binding site indicates a larger bias coefficient of the binding site.

12. The method according to any one of claims 1 to 11, wherein the ligand structure comprises a micromolecular ligand structure or a macromolecular ligand structure.

13. The method according to any one of claims 1 to 12, wherein the receptor structure comprises a protein structure.

14. A metadynamics-based target discovery apparatus, wherein the target discovery apparatus comprises a simulation unit, a determining unit, and an output unit, wherein
the simulation unit is configured to perform dynamics simulation on a ligand structure and a receptor structure, wherein a bias potential is configured for the ligand structure during the dynamics simulation, the bias potential of the ligand structure enables the ligand structure to traverse a surface of the receptor structure during the dynamics simulation, a calculation model for the bias potential comprises a bias coefficient expression, and the bias coefficient expression reflects a bias degree of a binding conformation obtained by binding the ligand structure to the receptor structure;
the determining unit is configured to determine target information of binding sites of the ligand structure on the surface of the receptor structure, wherein the target information comprises retention time or a bias coefficient, a bias coefficient of each binding site is calculated based on a target parameter and the bias coefficient expression, and the target parameter comprises a parameter value generated by the ligand structure during the dynamics simulation; and
the output unit is configured to output pocket information of the receptor structure based on a sorting result of the target information of the binding sites.

15. The apparatus according to claim 14, wherein
the output unit is further configured to output display information in a visual manner, wherein the display information comprises one or two of a process of traversing the surface of the receptor structure by the ligand structure during the dynamics simulation, or a process of binding the ligand structure to the receptor structure to form the binding conformation.

16. The apparatus according to claim 14 or 15, wherein in terms of outputting the pocket information of the receptor structure based on the sorting result of the target information of the binding sites, the output unit is specifically configured to:
use, as the pocket information of the receptor structure, binding sites corresponding to the first N pieces of target information in the sorting result of the target information of the binding sites, wherein N is a positive integer greater than or equal to 1.

17. The apparatus according to any one of claims 14 to 16, further comprising an extraction unit and an update unit, wherein
the determining unit is further configured to determine a solvent accessible surface of the receptor structure;
the extraction unit is configured to extract a plurality of discrete points from the solvent accessible surface; and
the update unit is configured to update location coordinates of a first discrete point based on one or more heavy atoms closest to the first discrete point, wherein the first discrete point is any one of the plurality of discrete points.

18. The apparatus according to claim 17, wherein in terms of updating the location coordinates of the first discrete point based on the one or more heavy atoms closest to the first discrete point, the update unit is specifically configured to:
map the first discrete point to a first coordinate system, and determine a second coordinate system based on a mapped location, wherein the first coordinate system is a coordinate system constructed based on locations of the first M heavy atoms closest to the first discrete point, the first discrete point is any one of the plurality of discrete points, and M is a positive integer greater than or equal to 3; and
update the location coordinates of the first discrete point based on the second coordinate system, wherein location coordinates of the binding sites are location coordinates of discrete points closest to the binding sites, and the location coordinates of the binding sites are used to determine bias potentials of the binding sites.

19. The apparatus according to claim 17 or 18, wherein in terms of extracting the plurality of discrete points from the solvent accessible surface, the extraction unit is specifically configured to:
extract the plurality of discrete points from the solvent accessible surface based on a density requirement.

20. The apparatus according to claim 18 or 19, wherein the output unit is further configured to output the plurality of discrete points in a visual manner.

21. The apparatus according to any one of claims 14 to 20, wherein the calculation model for the bias potential is used to constrain, by using the bias coefficient expression and the target parameter, an acting force applied to the ligand structure.

22. The apparatus according to claim 21, wherein the target parameter further comprises a preset parameter, the parameter value generated during the dynamics simulation comprises center-of-mass location coordinates of the ligand structure and location coordinates of the discrete points, and the preset parameter comprises a height of a Gaussian peak, a Gaussian of full width at half maximum, and a metadynamics harmonic parameter.

23. The apparatus according to claim 21 or 22, wherein the target parameter further comprises a confining potential parameter, the confining potential parameter is used to constrain a distance from the ligand structure to the surface of the receptor structure to be less than a preset threshold, and the confining potential parameter comprises a spring constant and the distance from the ligand structure to the surface of the receptor structure.

24. The apparatus according to any one of claims 14 to 23, wherein during the dynamics simulation, longer retention time of the ligand structure at the binding site indicates a larger bias coefficient of the binding site.

25. The apparatus according to any one of claims 14 to 24, wherein the ligand structure comprises a micromolecular ligand structure or a macromolecular ligand structure.

26. The apparatus according to any one of claims 14 to 25, wherein the receptor structure comprises a protein structure.

27. An electronic device, wherein the electronic device comprises at least one processor and a communication interface, wherein
the communication interface is configured to receive and/or send data, and/or the communication interface is configured to provide input and/or output for the processor; and
the at least one processor is configured to implement the method according to any one of claims 1 to 13.

28. A computer-readable storage medium, wherein the computer-readable storage medium stores instructions, and when the instructions are run on at least one processor, the method according to any one of claims 1 to 13 is implemented.
